# EUROPEAN PATENT APPLICATION

(11) **EP 4 674 425 A1**
(43) Date of publication of application: **07.01.2026**
(21) Application number: 24764232.5
(22) Date of filing: 07.02.2024
(51) Int. Cl.: A61K 38/08, A61P 27/02

(54) **COMPOSITION FOR TREATING DIABETIC RETINOPATHY, COMPRISING PEPTIDE**

(30) Priority: 27.02.2023 KR 20230025972
(71) Applicant: EyebioKorea, Inc., Busan 47397 (KR)
(72) Inventor: CHO, Yun Seok, Gyeonggi-do 16323 (KR); AHN, Byul Nim, Busan 47327 (KR); LEE, Yu Jin, Busan 47241 (KR)
(74) Representative: Hindles Limited
(86) International application number: PCT/KR2024/095091
(87) International publication number: WO 2024/181833

(57) **Abstract**

The present invention relates to a composition for preventing or treating diabetic retinopathy, comprising a peptide, wherein the peptide exhibits excellent inhibition with respect to vasodilation, loss of pericytes, and generation of neovascularity in the retina, and thus can be effectively used for preventing or treating retinopathy by inhibiting an increase in vascular permeability, inflammation, and the occurrence of neovascularity.

## Description

### Title of Invention

### COMPOSITION FOR TREATING DIABETIC RETINOPATHY, COMPRISING PEPTIDE

### Technical Field

The present invention relates to a composition for preventing or treating diabetic retinopathy, comprising a peptide consisting of 7 amino acids. Specifically, the peptide of the present invention is a peptide consisting of the amino acid sequence of SEQ ID NO: 1, and has an excellent effect in the treatment of retinopathy by inhibiting the generation of neovascularity in the retina, the loss of pericytes, and the vasodilation.

### Background Art

Diabetic retinopathy is one of the three major microvascular complications of diabetes mellitus, and is a disease in which persistent hyperglycemia and metabolic abnormalities cause damage to capillaries, resulting in damage, inflammation, and neovascularity throughout the retina, resulting in visual impairment.

Diabetic retinopathy, which occurs in about 60-70% of patients with diabetes mellitus for about 15 years, is divided into nonproliferative diabetic retinopathy (NPDR) and proliferative diabetic retinopathy (PDR) depending on symptoms and progression.

Retinal blood vessels mainly have a blood-retinal barrier (BRB) structure with selective permeability and protect retinal neurons from external substances. In the early stage of diabetic retinopathy, tight junctions and glia around blood vessels are damaged by hyperglycemia, damaging the blood-retinal barrier and causing blood components to leak into the retina, resulting in microvascular abnormalities in the retina such as hard exudates, retinal edema, and vascular abnormalities. In addition, plasma from blood vessels due to microvascular disorders and occlusion can cause retinal edema in the macula, leading to decreased visual acuity.

Proliferative diabetic retinopathy (PDR), the later stage of diabetic retinopathy, is a condition in which the generation of neovascularity in the retina can cause serious visual impairment. Manifestation such as retinal neovascularity and fibrous tissue proliferation is observed. New blood vessels originate from blood vessels in the retina and proliferate toward the back of the vitreous body. If bleeding occurs from these new blood vessels, it can cause serious decreased visual acuity and blindness, so immediate treatment such as intravitreal injection of anti-vascular endothelial growth factor (anti-VEGF) is required.

Treatment of diabetic retinopathy includes risk factor control, laser treatment, intraocular injection treatment, surgical treatment (vitrectomy), and the like. Recently, methods of injecting anti-vascular endothelial growth factor (anti-VEGF) such as aflibercept (Eylea), ranibizumab (Lucentis), and bevacizumab (Avastin) have been used, but these have disadvantages such as occurrence of side effects, high cost, repeated administration, and risk of rapid deterioration when the injection is discontinued.

Accordingly, the present inventors continued to study the treatment of eye diseases targeting peptides that have advantages such as easy mass production and excellent bioavailability. As a result, the present inventors confirmed a peptide that has an excellent effect in the treatment of diabetic retinopathy. Based on the above, the present inventors completed the present invention.

### Prior Art Document

### Patent Document

(Patent Document 1) Korean Patent Application Publication No. 10-2020-0134175

### Detailed Description of Invention

### Technical Problem

An object of the present invention is to provide a pharmaceutical composition for preventing or treating retinopathy, comprising a peptide consisting of to the amino acid sequence of SEQ ID NO: 1, or a sequence having 90% or more homology to the amino acid sequence of SEQ ID NO: 1.

In addition, another object of the present invention is to provide a pharmaceutical composition for preventing or treating nonproliferative diabetic retinopathy (NPDR) and proliferative diabetic retinopathy (PDR), comprising a peptide consisting of the amino acid sequence of SEQ ID NO: 1.

### Solution to Problem

The present invention provides a pharmaceutical composition for preventing or treating retinopathy, comprising a peptide consisting of the amino acid sequence of SEQ ID NO: 1, or a sequence having 80%, 90%, 95%, 97%, 98%, or 99% or more homology to the amino acid sequence of SEQ ID NO: 1.

The peptide can inhibit the generation of neovascularity, the loss of pericytes, or the vasodilation, and can inhibit the expression of vascular endothelial growth factor, the phosphorylation of Akt (protein kinase B), the expression of COX-2, or the phosphorylation of JNK (c-Jun N-terminal kinase), and can restore or improve ZO-1, a tight junction protein.

The retinopathy may be diabetic retinopathy, retinopathy of prematurity, diabetic retinal edema, or retinal vein occlusion, and preferably diabetic retinopathy.

The diabetic retinopathy may be nonproliferative diabetic retinopathy or proliferative diabetic retinopathy.

The present invention provides a composition for use in the prevention or treatment of retinopathy, comprising a peptide consisting of the amino acid sequence of SEQ ID NO: 1, or a sequence having 80%, 90%, 95%, 97%, 98%, or 99% or more homology to the amino acid sequence of SEQ ID NO: 1.

The present invention provides a use of the composition for the prevention or treatment of retinopathy, comprising a peptide consisting of the amino acid sequence of SEQ ID NO: 1, or a sequence having 80%, 90%, 95%, 97%, 98%, or 99% or more homology to the amino acid sequence of SEQ ID NO: 1.

The present invention provides a method for treating retinopathy by administering to a subject having retinopathy a peptide consisting of the amino acid sequence of SEQ ID NO: 1, or a sequence having 80%, 90%, 95%, 97%, 98%, or 99% or more homology to the amino acid sequence of SEQ ID NO: 1.

### Effects of Invention

The peptide of the present invention exhibits excellent inhibition with respect to vasodilation, loss of pericytes, and generation of neovascularity in the retina, and thus can be effectively used for preventing or treating retinopathy by inhibiting an increase in vascular permeability, inflammation, and the occurrence of neovascularity.

### Brief Description of Drawings

Figures 1 and 2 show the fluorescence images of retinal blood vessels in an oxygen induced retinopathy mouse model according to the administration of the peptide of the present invention, and the results obtained by calculating the number and area of new blood vessels.
Figure 3 shows the results obtained by electroretinography in a streptozotocin induced diabetic retinopathy mouse model according to the administration of the peptide of the present invention.
Figure 4 shows the results obtained by electroretinography in a db/db mouse model according to the administration of the peptide of the present invention.
Figures 5 and 6 show the PAS staining images of retinal blood vessels in a db/db mouse model according to the administration of the peptide of the present invention, and the results obtained by quantifying the number of vascular endothelial cells and pericytes.
Figure 7 shows the results obtained by confirming the expression of proteins in the retinal tissue in a streptozotocin induced diabetic retinopathy mouse model according to the administration of the peptide of the present invention.
Figure 8 shows the results obtained by confirming the expression of vascular endothelial growth factor (VEGF) in the retinal tissue in a db/db mouse model according to the administration of the peptide of the present invention.
Figure 9 shows the results obtained by confirming the phosphorylation level of Akt in the retinal tissue in a db/db mouse model according to the administration of the peptide of the present invention.
Figure 10 shows the results obtained by confirming the expression of COX-2 in the retinal tissue in a db/db mouse model according to the administration of the peptide of the present invention.
Figure 11 shows the results obtained by confirming the phosphorylation level of JNK in the retinal tissue in a db/db mouse model according to the administration of the peptide of the present invention.
Figures 12 and 13 show the fluorescence images of retinal blood vessels (*: optic nerve) in a zebrafish diabetic retinopathy model according to the administration of the peptide of the present invention, and the results obtained by calculating the vascular diameter.
Figures 14 and 15 show the results obtained by confirming the expression of genes in the retinal tissue in a zebrafish diabetic retinopathy model according to the administration of the peptide of the present invention.
Figures 16 and 17 show the fluorescence images of the retinal tissue in a zebrafish diabetic retinopathy model according to the administration of the peptide of the present invention, and the results obtained by confirming the expression of cells and proteins.

### Best Mode for Carrying out the Invention

Hereinafter, with reference to the accompanying drawings, embodiments and examples of the present invention will be described in detail so that those of ordinary skill in the art to which the present invention belongs can easily practice the present invention. However, the present invention may be implemented in various forms and is not limited to the embodiments and examples described herein.

Throughout the present specification, when a certain part "includes" a certain component, it means that other components may be further included, rather than excluding other components, unless otherwise stated.

The present invention relates to a composition for preventing, improving, or treating retinopathy, comprising a peptide consisting of the amino acid sequence of SEQ ID NO: 1.

The composition may be a pharmaceutical composition for preventing or treating diabetic retinopathy.

The amino acid sequence of SEQ ID NO: 1 may be a sequence consisting of "Hyp-Gly-Gln-Glu-Aib-Leu-Ala", or may comprise a sequence that is 90% or more, 93% or more, 95% or more, 96% or more, 97% or more, 98% or more, or 99% or more identical to the amino acid sequence of SEQ ID NO: 1.

In the present invention, Hyp is trans-4-hydroxy-L-proline,
Gly is glycine,
Gln is glutamine,
Glu is glutamate or glutamic acid,
Aib is 2-aminoisobutyric acid,
Leu is leucine, and
Ala is alanine.

In the present invention, "diabetic retinopathy" refers to an eye complication in which peripheral circulation disorder occurs due to diabetes mellitus and microcirculation of the retina is disturbed, resulting in decreased visual acuity. Initially, it may not cause any symptoms or may only cause mild visual acuity problems, but it may eventually lead to blindness. Diabetic retinopathy can occur in anyone with type 1 diabetes mellitus or type 2 diabetes mellitus.

In the present invention, the diabetic retinopathy may be nonproliferative diabetic retinopathy (NPDR) or proliferative diabetic retinopathy (PDR), but is not limited thereto.

As used herein, the term "neovascularization" refers to the process of forming new blood vessels, that is, the development of new blood vessels into cells, tissues, or organs, and "neovascularity" refers to blood vessels newly created through the neovascularization process. As used herein, "neovascularization" and "neovascularity" can be used interchangeably.

As used herein, the term "prevention" refers to any action of inhibiting or delaying the onset of a gastrointestinal disease and the like by administration of the composition according to the present invention, and "treatment" refers to any action in which symptoms of a subject suspected of and suffering from a gastrointestinal disease are improved or beneficially changed by administration of the composition. As used herein, the term "improvement" refers to any action of at least reducing the parameters related to the condition being treated, such as the degree of symptoms, by administration of the composition comprising the extract of the present invention.

Hereinafter, the present invention will be described in more detail through the examples, but the following examples are for illustrative purposes only and are not intended to limit the scope of the present invention.

### [Preparation Example]

### Preparation of a peptide consisting of 7 amino acids (7 mer)

The peptide of the present invention was synthesized based on the previously known solid phase peptide synthesis (SPPS) method, and the manufacturing process included the following steps.
Step 1: resin soaking and loading
Step 2: solid phase peptide synthesis (SPPS)
Step 3: deprotected peptide synthesis (global cleavage)
Step 4: primary purification and concentration / secondary purification and concentration
Step 5: freeze drying

The solid phase peptide synthesis (SPPS) method includes the following steps: loading a first amino acid into a resin, deprotecting the N-terminus of the amino acid with Fmoc, and then performing amino acid coupling according to the amino acid sequence, and synthesizing the protected peptide in a solid phase reactor. In addition, the amino acid coupling includes the following steps: loading a first amino acid into a resin and then removing Fmoc from the N-terminus of the amino acid; after completing the reaction, removing the solvent and washing the resin; coupling the next amino acid according to the sequence; after completing the reaction, removing the solvent and washing the resin; and repeating the above process until the final amino acid sequence was generated.

The alpha amine group of each amino acid is protected with a base-labile Fmoc group, and the functional group of the side chain is protected with an acid-labile group. All amino acids except Gly and Aib are in the L-configuration, and among them, several amino acids such as Hyp (tBu), Glu (tBu), and Gln (Trt) have unique protecting group. In addition, amino acids such as Ala, Leu, Aib, and Gly do not have a protecting group of the side chain. Therefore, after completing amino acid coupling according to the sequence, the resin and the protecting group were removed from the peptide to obtain a crude peptide. Thereafter, purification, concentration, and freeze-drying were performed to obtain the peptide compounds consisting of the amino acid sequence of SEQ ID NO: 1.

### [Example 1]

### Evaluation of the efficacy of inhibiting neovascularity in an oxygen induced retinopathy mouse model

In order to evaluate the efficacy of the peptide of the present invention in inhibiting neovascularity in an oxygen induced retinopathy (OIR) mouse model, an experiment was performed as follows.

The oxygen induced retinopathy (OIR) mouse model is a model of ischemic retinopathy such as proliferative diabetic retinopathy (PDR), retinopathy of prematurity (ROP), and retinal vein occlusion (RVO), and is a model that exhibits active generation of neovascularity in the retina.

After stabilizing the mice born with the mother (new born mice) for about 3 to 4 days, they were raised under 75% hyperoxic conditions for 5 days from day 7 to day 11 after birth. Thereafter, they were raised under normoxic conditions until day 17 after birth. Drugs were administered while they were raised under normoxic conditions. The peptide of the present invention (002-175) was administered as eye drops (ED) for 5 days from day 12 to day 16 after birth, and as a positive control group, aflibercept was administered intraocularly (intravitreal injection, IVT) once on day 12 after birth. Thereafter, the mouse eyes were extracted, fixed in 10% formalin for 1 hour, and then flat mounted. The retina was divided into four equal parts centered on the optic nerve, and treated with Isolectin B4 (1 mM CaCl₂, 1:200) overnight (O/N). Thereafter, they were spread on a slide glass, sprayed with mounting solution, covered with a cover glass, and photographed under a fluorescence microscope. The fluorescence images of blood vessels in the retina were obtained, and the number and area of new blood vessels and the area of blood vessels were calculated using the imageJ program.

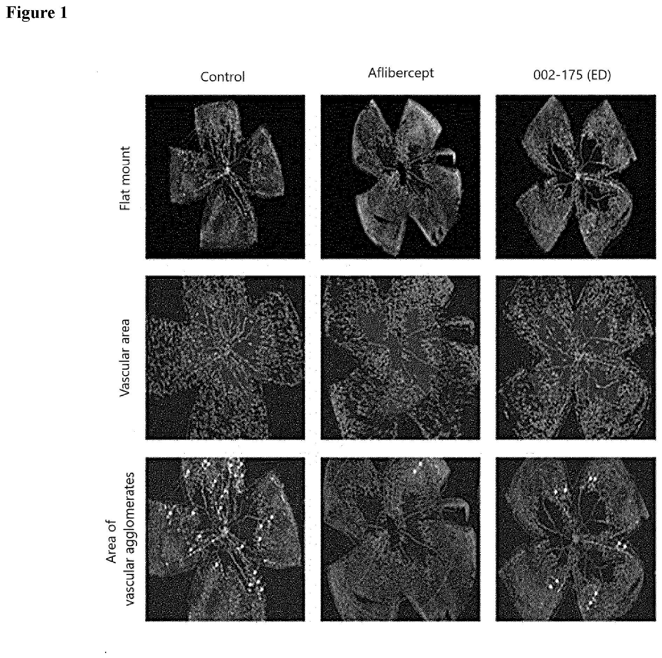

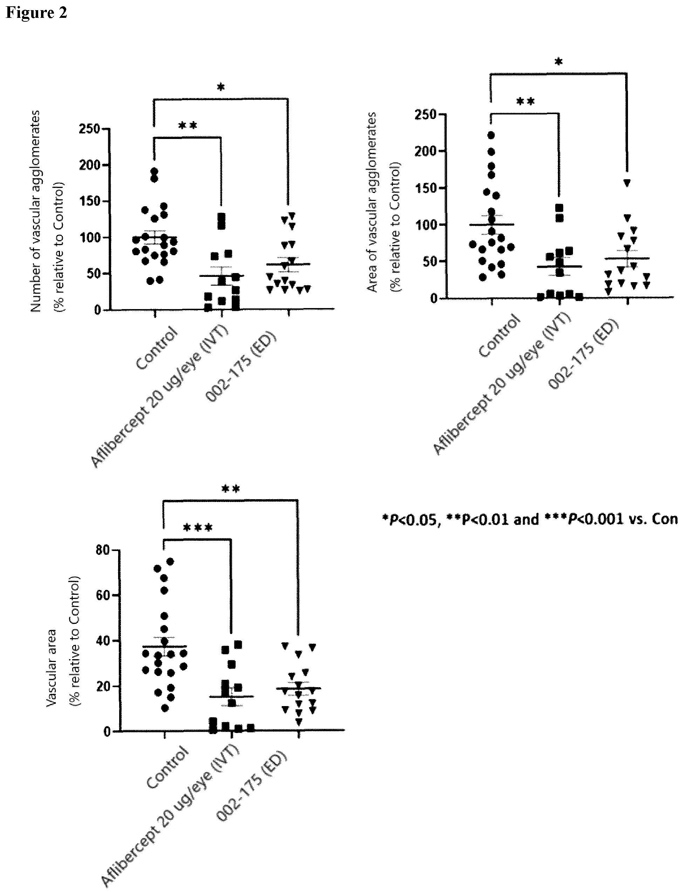

As a result, as shown in Figures 1 and 2, the oxygen induced retinopathy mouse model showed the area of new blood vessels, while the group administered with the peptide of the present invention showed a decrease in the area of new blood vessels to a level similar to that of the group administered with aflibercept.

Therefore, it can be seen that the peptide of the present invention has the efficacy of inhibiting neovascularity.

### [Example 2]

### Evaluation of the efficacy of inhibiting neovascularity in a high glucose induced diabetic retinopathy mouse model

In order to evaluate the efficacy of the peptide of the present invention in inhibiting neovascularity in a high glucose induced diabetic retinopathy mouse model, an experiment was performed as follows.

### 2-1. Streptozotocin (STZ) induced diabetic retinopathy mouse model

The streptozotocin induced mouse model is a type 1 diabetes mellitus model that induces diabetes mellitus by injecting the antibiotic streptozotocin (STZ) into the mouse's peritoneum to damage the beta cells that produce insulin in the pancreas. It is one of the models used in diabetic retinopathy research, and it is a model that can confirm the early stage of diabetic retinopathy from the time of onset of diabetes mellitus.

After fasting for 3-4 hours before administration, 8-week-old C57BL/6 mice were administered with streptozotocin (STZ) dissolved in Na-citrate buffer and buffer at a dose of 50 mg/kg intraperitoneally for 5 days. The drug used at this time was prepared immediately before administration and used quickly. One week after the last STZ intraperitoneal administration, blood glucose was measured, and only mice with blood glucose exceeding 250 mg/dL were determined to be diabetic mice and used in the experiment. The drug was administered as eye drops (ED) twice a day for about 17 weeks starting from the day after model production, and 10 µg was administered intraocularly (intravitreal injection, IVT) once a month. In order to evaluate retinal function, the mice were dark-adapted for more than 12 hours before measuring electroretinogram (ERG), and then the mice were subjected to pupillary dilation and anesthetized, and electrodes were placed on the skin, tail, and cornea to measure ERG. The retina was stimulated with a single white light to obtain a response value, and the amplitude from the trough of the a wave to the peak of the b wave was measured, which was evaluated as an index of retinal function.

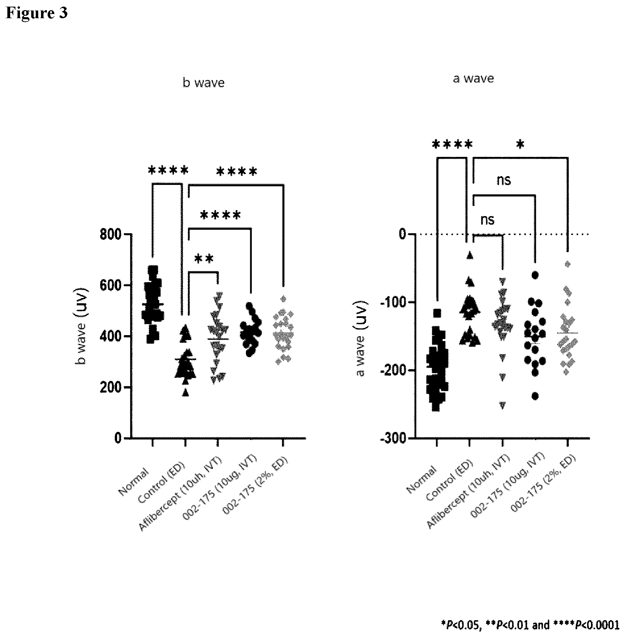

As a result, as shown in Figure 3, the STZ induced diabetic retinopathy mouse group showed a decrease in the amplitude of the a wave and the b wave, whereas the group administered with the peptide of the present invention showed an increase in the amplitude of the a wave and the b wave to a level similar to that of the group administered with aflibercept.

Therefore, it can be seen that the peptide of the present invention has the efficacy of inhibiting neovascularity.

### 2-2. db/db mouse model

The db/db mouse model is a mouse with a mutation in the receptor of leptin, an appetite-suppressing hormone, and is widely used as a type 2 diabetes mellitus model because it exhibits phenotypes such as severe obesity, hyperphagia, insulin resistance, and diabetes mellitus, and is a model that can confirm the early stage of diabetic retinopathy.

The drug was administered as eye drops (ED) to 12-week-old db/db mice twice a day (BID) or three times a day (TID) for about 12 weeks. In order to evaluate retinal function, the mice were dark-adapted for more than 12 hours before measuring electroretinogram (ERG), and then the mice were subjected to pupillary dilation and anesthetized, and electrodes were placed on the skin, tail, and cornea to measure ERG. The retina was stimulated with a single white light to obtain a response value, and the amplitude from the trough of the a wave to the peak of the b wave was measured, which was evaluated as an index of retinal function.

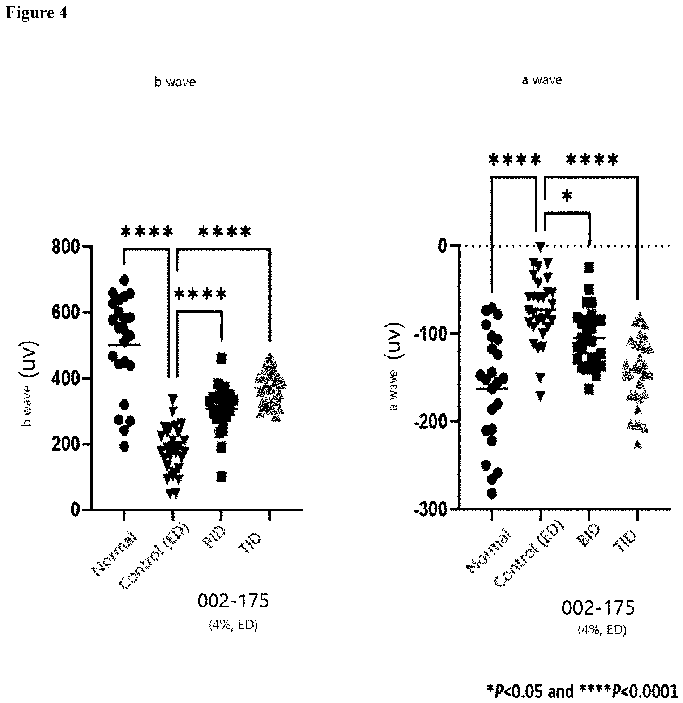

As a result, as shown in Figure 4, the db/db mouse group showed a decrease in the amplitude of the a wave and the b wave, whereas the group administered with the peptide of the present invention showed an increase in the amplitude of the a wave and the b wave. In particular, it was confirmed that the group administered three times a day (TID) showed a more excellent effect compared to the group administered twice a day (BID).

Therefore, it can be seen that the peptide of the present invention has the efficacy of inhibiting neovascularity.

### [Example 3]

### Evaluation of the efficacy of inhibiting loss of pericytes in a high glucose induced diabetic retinopathy mouse model

In the early stage of nonproliferative diabetic retinopathy, blood flows into the tissue due to the loss of pericytes surrounding the capillaries, causing the macula to swell. Macular edema is a representative symptom of nonproliferative diabetic retinopathy in which the focus of objects becomes blurred, the field of vision darkens, and visual acuity temporarily decreases. Therefore, in order to evaluate the efficacy of the peptide of the present invention in inhibiting loss of pericytes, an experiment was performed as follows.

The drug was administered as eye drops (ED) to 12-week-old db/db mice twice a day (BID) or three times a day (TID) for about 12 weeks. In order to evaluate the state of blood vessels in the retina, the eyes of the extracted mice were fixed in 10% formalin for a week. Thereafter, the fixed eye tissue was treated with elastase to separate the retinal blood vessels and PAS staining was performed. The ratio of vascular endothelial cells and pericytes present in the blood vessel walls and the number of acellular blood vessels were quantified to evaluate the permeability of the blood vessels in the retina.

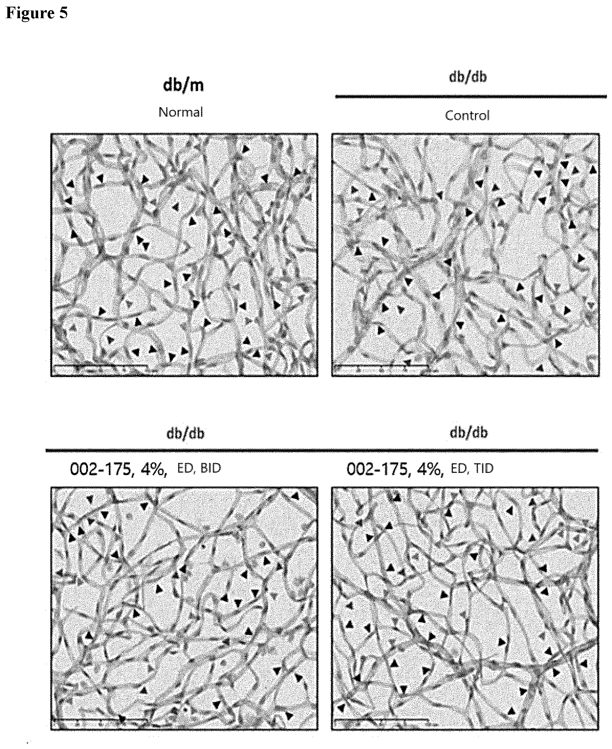

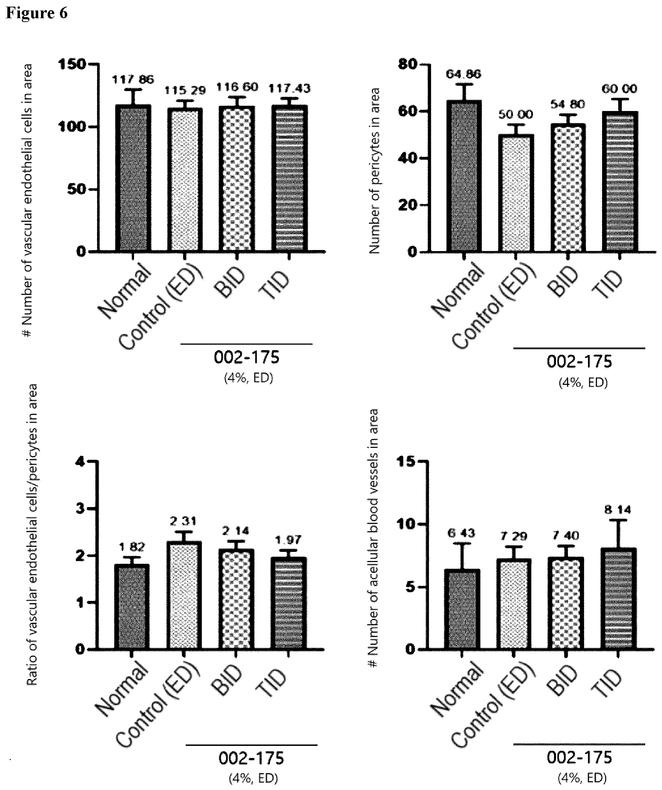

As a result, as shown in Figures 5 and 6, the db/db mouse group showed a decrease in pericytes, whereas the group administered with the peptide of the present invention showed an inhibition of the decrease in pericytes. In particular, it was confirmed that the group administered three times a day (TID) showed a more excellent effect compared to the group administered twice a day (BID).

Therefore, it can be seen that the peptide of the present invention has the efficacy of inhibiting the loss of pericytes.

### [Example 4]

### Evaluation of the expression level of proteins in the retina in a high glucose induced diabetic retinopathy mouse model

In order to evaluate the expression level of proteins in the retinal tissue in a high glucose induced diabetic retinopathy mouse model according to the administration of the peptide of the present invention, an experiment was performed as follows.

### 4-1. Streptozotocin (STZ) induced diabetic retinopathy mouse model

After fasting for 3-4 hours before administration, 8-week-old C57BL/6 mice were administered with streptozotocin (STZ) dissolved in Na-citrate buffer and buffer at a dose of 50 mg/kg intraperitoneally for 5 days. The drug used at this time was prepared immediately before administration and used quickly. One week after the last STZ intraperitoneal administration, blood glucose was measured, and only mice with blood glucose exceeding 250 mg/dL were determined to be diabetic mice and used in the experiment. The drug was administered as eye drops (ED) twice a day for about 17 weeks starting from the day after model production, and 10 µg was administered intraocularly (intravitreal injection, IVT) once a month. In order to evaluate the expression level of proteins in the retina, retinal tissue was separated from the extracted mouse eyes and treated with RIPA solution to extract proteins in the retina. Western blot was performed to evaluate the expression level of factors related to neovascularization and vascular permeability in the retina.

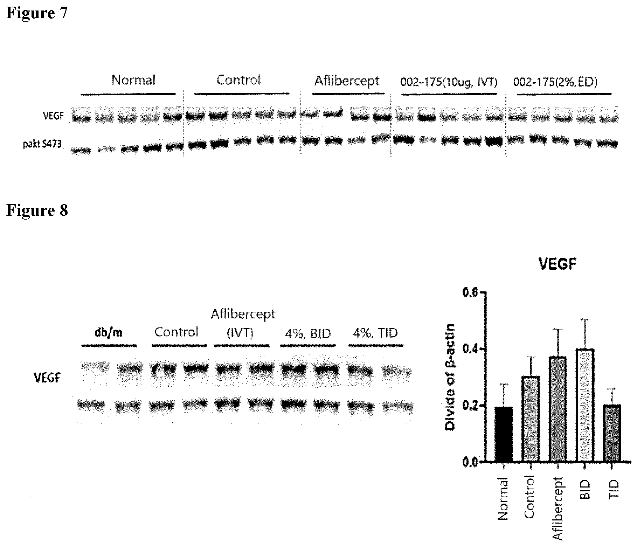

As a result, as shown in Figure 7, the STZ induced diabetic retinopathy mouse group showed an increase in the expression of vascular endothelial growth factor (VEGF), which is related to the generation of neovascularity, whereas the group administered with the peptide of the present invention showed a decrease in the expression of vascular endothelial growth factor.

In addition, the STZ induced diabetic retinopathy mouse group showed an increase in the phosphorylation level of Akt, a signal transduction factor related to vascular endothelial growth factor, whereas the group administered with the peptide of the present invention showed a decrease in the phosphorylation level of Akt.

Therefore, it can be seen that the peptide of the present invention inhibits the expression of proteins related to the permeability of new blood vessels and vascular cells in the retina.

### 4-2. db/db mouse model

The drug was administered as eye drops (ED) to 12-week-old db/db mice twice a day (BID) or three times a day (TID) for about 12 weeks. In order to evaluate the expression level of proteins in the retina, retinal tissue was separated from the extracted mouse eyes and treated with RIPA solution to extract proteins in the retina. Western blot was performed to evaluate the expression level of factors related to neovascularization and vascular permeability in the retina.

As a result, as shown in Figure 8, the db/db mouse group showed an increase in the expression of vascular endothelial growth factor (VEGF), whereas the group administered with the peptide of the present invention three times a day (TID) showed a decrease in the expression of vascular endothelial growth factor.

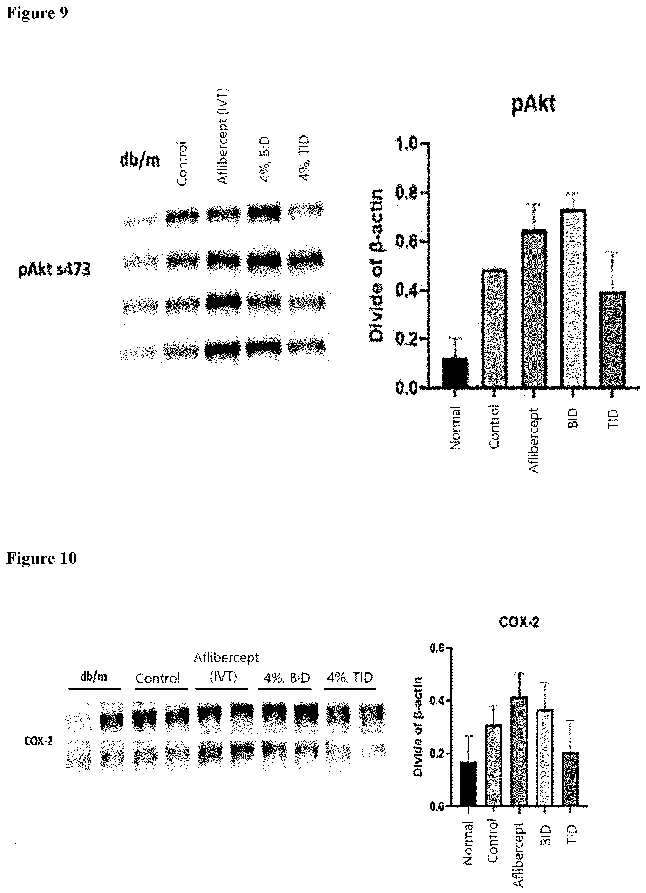

In addition, as shown in Figure 9, the db/db mouse group showed an increase in the phosphorylation level of Akt, whereas the group administered with the peptide of the present invention three times a day (TID) showed a decrease in the phosphorylation level of Akt.

In addition, as shown in Figure 10, the db/db mouse group showed an increase in the expression of COX-2, which is a factor related to inflammation, whereas the group administered with the peptide of the present invention three times a day (TID) showed a decrease in the expression of COX-2.

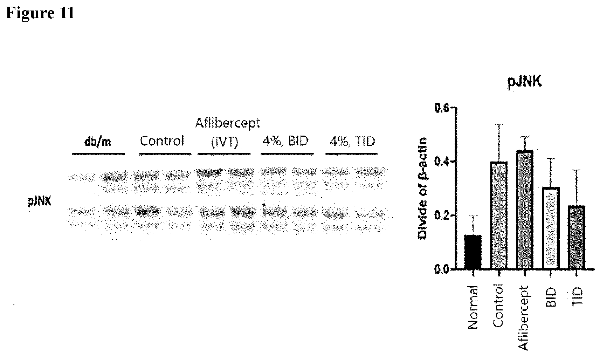

In addition, as shown in Figure 11, the db/db mouse group showed an increase in the phosphorylation level of JNK, which is a factor related to cell death, whereas the group administered with the peptide of the present invention showed a decrease in the phosphorylation level of JNK.

Therefore, it can be seen that the peptide of the present invention inhibits the expression of proteins related to the permeability of new blood vessels and vascular cells in the retina, and inhibits the expression of factors related to inflammation.

### [Example 5]

### Evaluation of the efficacy of inhibiting retinal vasodilation in a zebrafish diabetic retinopathy model

Among the embryos obtained by mating adult zebrafish Tg[flk:EGFP], embryos expressing a fluorescent protein were selected and used. On the 6th day after fertilization, the embryos were treated with glucose and the peptide of the present invention (002-175) for 4 days, and then euthanized by adding 4 mg/ml of Tricane. After fixing with 4% paraformaldehyde for 3 days, the eyeballs were separated using 3% trypsin, and the changes in the blood vessels in the separated eyeballs were observed using a fluorescence microscope.

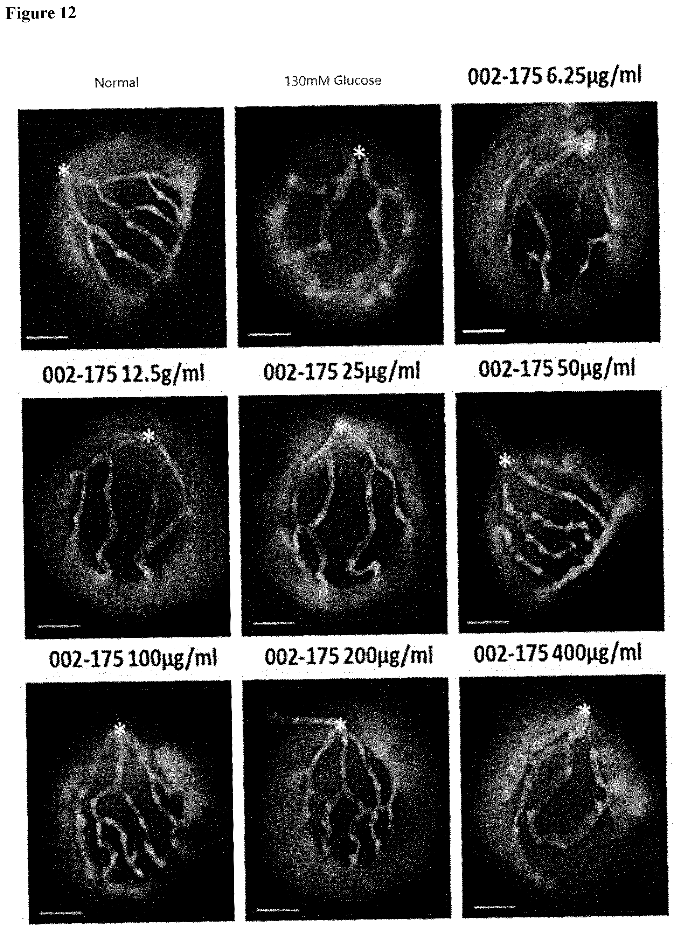

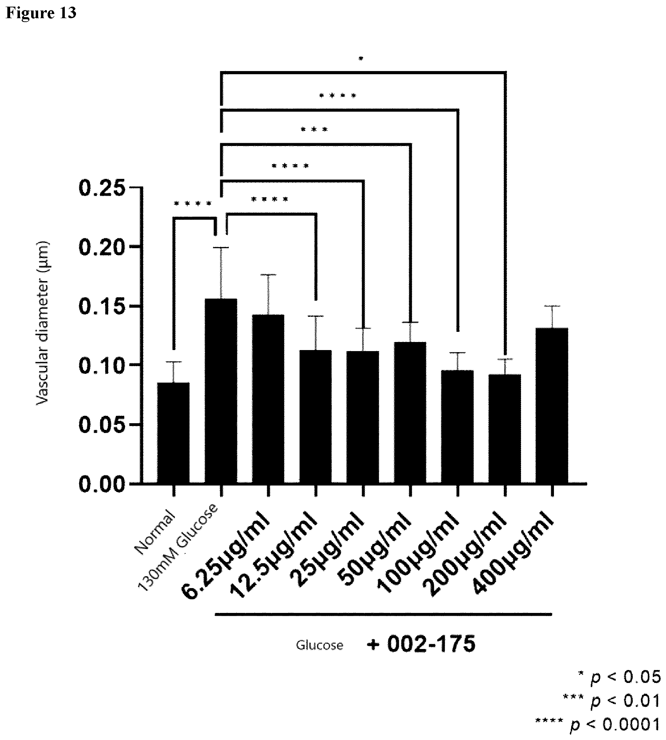

As a result, as shown in Figures 12 and 13, the group treated with high glucose (130 mM glucose) showed an increase in the diameter of zebrafish retinal blood vessels by more than 1.5 times compared to the normal group, whereas the group treated with the peptide of the present invention showed a decrease in the diameter of retinal blood vessels. In particular, the groups treated with 100 and 200 µg/ml showed a decrease in the diameter of retinal blood vessels to a level similar to that of the normal group.

Therefore, it can be seen that the peptide of the present invention has the efficacy of inhibiting retinal vasodilation.

### [Example 6]

### Analysis of gene expression in a zebrafish diabetic retinopathy model

Among the embryos obtained by mating adult zebrafish Tg[flk:EGFP], embryos expressing a fluorescent protein were selected and used. On the 6th day after fertilization, the embryos were treated with glucose and the peptide of the present invention (002-175) for 4 days, and then euthanized by adding 4 mg/ml of Tricane. The eyeballs were extracted, and RNA was extracted and analyzed using microarray. In addition, Trizol was added to the extracted eyeballs, and the tissue was homogenized to extract mRNA. The extracted mRNA was quantified using a NanoDrop spectrophotometer, and cDNA was synthesized using RNA with a purity of 1.8 or higher. The synthesized cDNA was subjected to polymerization reaction of SYBR Green Master Mix, primers, and genes, and the concentration of each gene was divided by GAPDH to obtain the corrected concentration of the gene.

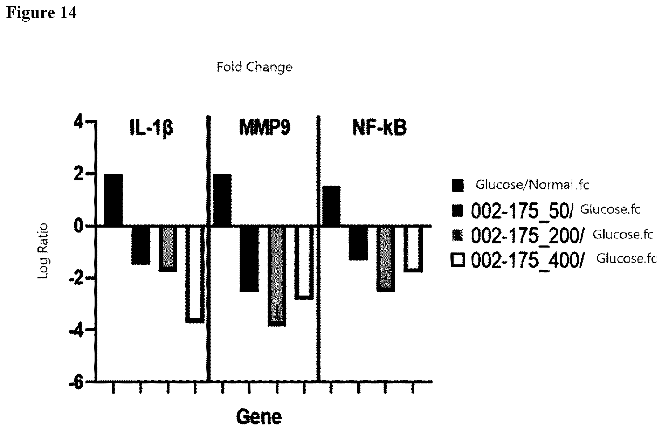

As a result, as shown in Figure 14, the analysis results through microarray showed that the group treated with the peptide of the present invention showed a decrease in the gene expression of IL-1β, MMP9, and NF-kB.

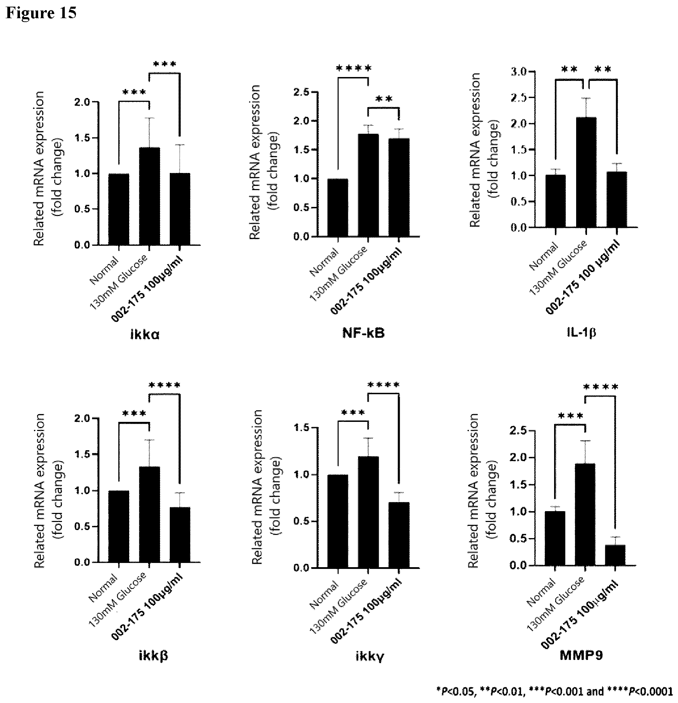

In addition, as shown in Figure 15, the real-time PCR result showed that the group treated with the peptide of the present invention showed a decrease in the gene expression of ikkα, ikkβ, ikkγ, NF-kB, IL-1β, and MMP9.

IL-1β is an inflammatory factor related to the generation of neovascularity, which increases vascular permeability and activates NF-kB in a hyperglycemic environment to promote the apoptosis of retinal capillary cells. In addition, NF-kB, which is present in the retina and microvessels, forms the central axis of the intracellular signaling system, and induces inflammatory-mediated apoptosis by increasing the expression of various cytokines such as IL-1β, IL-6, and IL-8 in the vitreous humor and serum. In addition, matrix metalloproteinases (MMPs) play an important role in the progression of diabetic retinopathy, and in particular, MMP9 is involved in the neovascularity of retinal capillary cells, and the activation of NF-kB, TNF-α, and interleukin increases MMP9, leading to the development of diabetic retinopathy.

Therefore, it can be seen that the peptide of the present invention has the efficacy of inhibiting inflammatory factors in the retina and the mechanism of diabetic retinopathy development.

### [Example 7]

### Analysis of histological changes in a zebrafish diabetic retinopathy model

On the 6th day after fertilization (6dpf, 6 days post-fertilization), zebrafish fry were fixed with 4% paraformaldehyde for 3-4 hours, and then treated with 30% sucrose, and treated overnight at 4°C. O.C.T blocks were made, and sections were performed, and then TUNEL staining and immunofluorescence (IF) staining were performed.

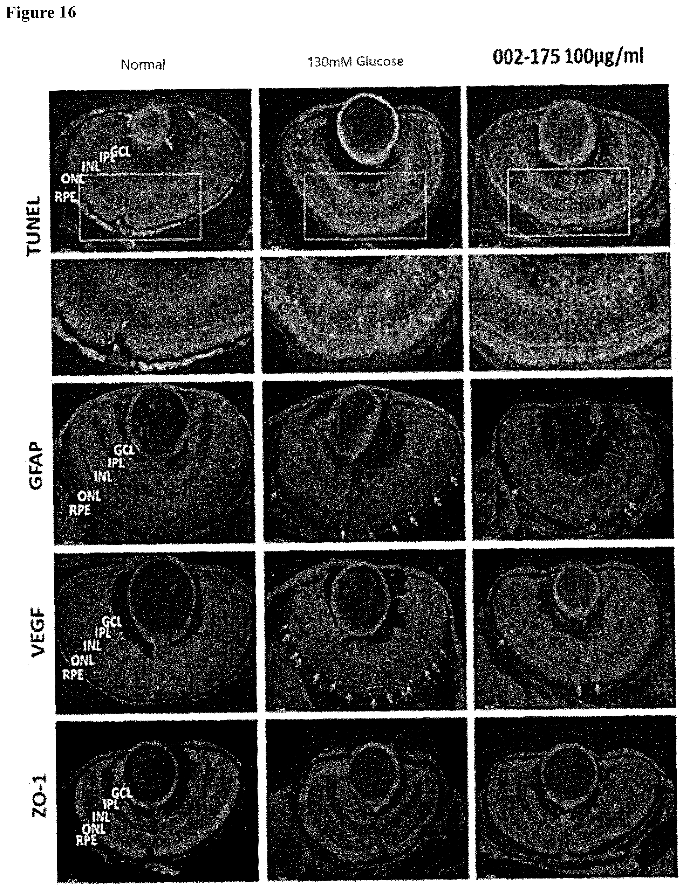

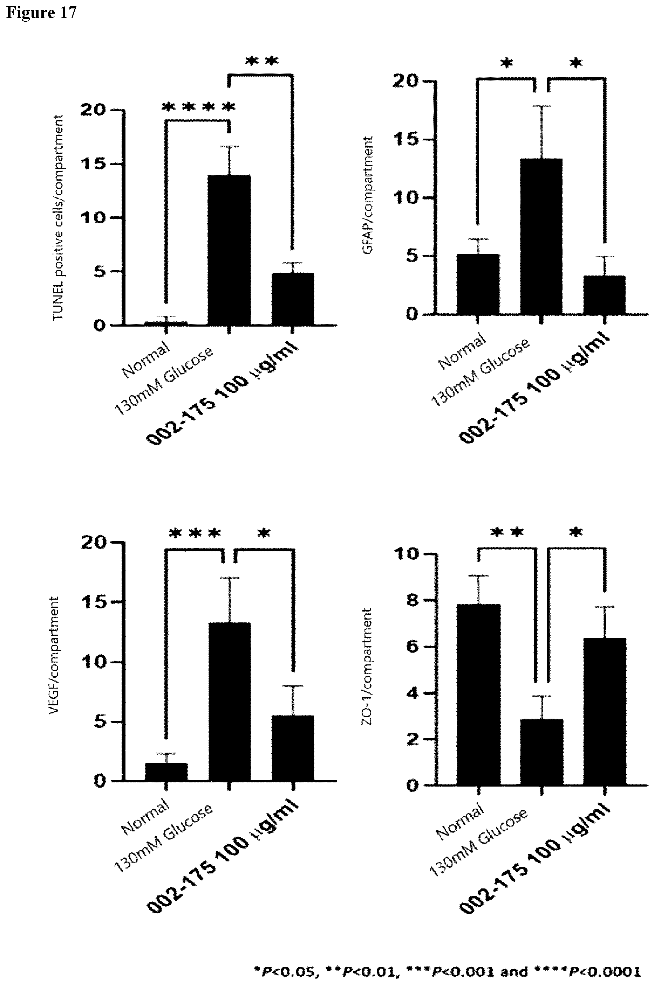

As a result, as shown in Figures 16 and 17, the TUNEL assay results in retinal tissue showed that the group treated with high glucose showed an increase in TUNEL positive cells in the inner nuclear layer (INL) of the retina, whereas the group treated with the peptide of the present invention (002-175) showed a decrease in TUNEL positive cells.

In addition, as a result of analyzing the expression of VEGF, GFAP, and ZO-1 in the zebrafish eye through immunofluorescence (IF) staining, the group treated with high glucose showed an increase in the expression of VEGF and GFAP, which are inflammatory factors, in the outer nuclear layer (ONL) of the retina, whereas the group treated with the peptide of the present invention (002-175) showed a decrease in the expression of VEGF and GFAP. In addition, in the case of ZO-1, a tight junction protein, the group treated with high glucose showed a decrease in expression, whereas the group treated with the peptide of the present invention (002-175) showed an increase in expression and a recovery pattern.

Therefore, it can be seen that the peptide of the present invention has the efficacy of inhibiting inflammatory factors in the retina and the mechanism of diabetic retinopathy development. In particular, in the high glucose induced diabetic retinopathy zebrafish model, it was confirmed that the tight junction marker ZO-1 decreased, resulting in damage to the blood-retinal barrier (BRB), and vascular edema was observed due to increased vascular permeability. This is clinically the same mechanism as diabetic retinal edema, and it can be seen that the peptide of the present invention effectively inhibits diabetic retinopathy and diabetic retinal edema by reducing vascular permeability through inhibition of neovascularity and inflammatory factors in the retina.

## Claims

1. A pharmaceutical composition for preventing or treating retinopathy, comprising a peptide consisting of a sequence having 90% or more homology to the amino acid sequence of SEQ ID NO: 1.

2. The pharmaceutical composition for preventing or treating retinopathy according to claim 1, wherein the pharmaceutical composition comprises a peptide consisting of a sequence having 99% or more homology to the amino acid sequence of SEQ ID NO: 1.

3. The pharmaceutical composition for preventing or treating retinopathy according to claim 1, wherein the pharmaceutical composition comprises a peptide consisting of the amino acid sequence of SEQ ID NO: 1.

4. The pharmaceutical composition for preventing or treating retinopathy according to claim 1, wherein the retinopathy is at least one selected from the group consisting of diabetic retinopathy, retinopathy of prematurity, diabetic retinal edema, and retinal vein occlusion.

5. The pharmaceutical composition for preventing or treating retinopathy according to claim 1, wherein the retinopathy is diabetic retinopathy.

6. The pharmaceutical composition for preventing or treating retinopathy according to claim 5, wherein the diabetic retinopathy is nonproliferative diabetic retinopathy or proliferative diabetic retinopathy.

7. The pharmaceutical composition for preventing or treating retinopathy according to claim 1, wherein the peptide inhibits the generation of neovascularity.

8. The pharmaceutical composition for preventing or treating retinopathy according to claim 1, wherein the peptide inhibits the loss of pericytes.

9. The pharmaceutical composition for preventing or treating retinopathy according to claim 1, wherein the peptide inhibits the vasodilation.

10. The pharmaceutical composition for preventing or treating retinopathy according to claim 1, wherein the peptide inhibits at least one of the expression of vascular endothelial growth factor, the phosphorylation of Akt (protein kinase B), the expression of COX-2, and the phosphorylation of JNK (c-Jun N-terminal kinase).

11. The pharmaceutical composition for preventing or treating retinopathy according to claim 1, wherein the peptide restores ZO-1, a tight junction protein.
